# EUROPEAN PATENT APPLICATION

(11) **EP 4 847 022 A1**
(43) Date of publication of application: **02.12.2026**
(21) Application number: 24920162.5
(22) Date of filing: 10.12.2024
(51) Int. Cl.: A61K 31/405, A61P 1/16, A61P 3/00

(54) **AGENT FOR PREVENTING OR AMELIORATING METABOLIC DYSFUNCTION-ASSOCIATED STEATOTIC LIVER DISEASES**

(30) Priority: 25.01.2024 JP 2024009619
(71) Applicant: Tohoku University, Sendai-shi, Miyagi 980-8577 (JP)
(72) Inventor: ABE, Takaaki, Sendai-shi, Miyagi 980-8577 (JP); KASAHARA, Tomoko, Sendai-shi, Miyagi 980-8577 (JP); KAKAZU, Eiji, Ichikawa-shi, Chiba 272-8516 (JP); MINO, Masaaki, Ichikawa-shi, Chiba 272-8516 (JP)
(74) Representative: Script Intellectual Property LLP
(86) International application number: PCT/JP2024/043581
(87) International publication number: WO 2025/158806

(57) **Abstract**

An object of the present invention is to provide an agent for preventing or ameliorating MASLD, such as MASH, comprising, as an active ingredient, a low-molecular-weight compound that can be produced relatively easily and inexpensively. An agent comprising one or more compounds selected from the group consisting of compounds of the following formula (Io) and physiologically acceptable salts thereof is defined as an agent for preventing or ameliorating MASLD: 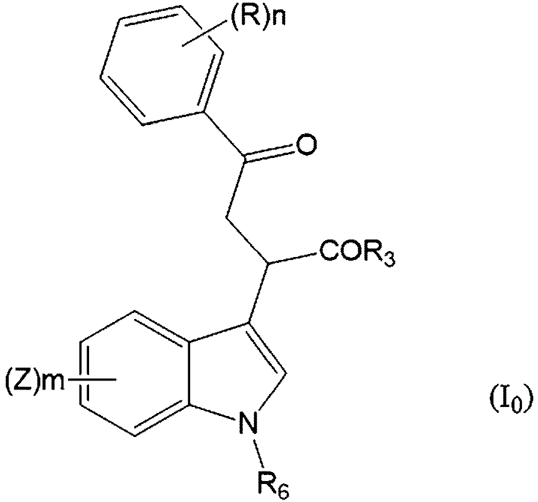 [wherein R is a halogen atom, an alkyl group having 1 to 7 carbon atoms, or an alkoxyl group having 1 to 7 carbon atoms, n is an integer of any one of 0 to 3; Z is a halogen atom, an alkyl group having 1 to 6 carbon atoms, an alkenyl group having 2 to 6 carbon atoms, an alkynyl group having 2 to 6 carbon atoms, or an organic oxy group of OR⁸, m is an integer of any one of 0 to 4, R⁸ represents an alkyl group having 1 to 7 carbon atoms, an alkenyl group having 2 to 6 carbon atoms, or an alkynyl group having 2 to 6 carbon atoms; when n and m are plural, each R and each Z are the same or different; R⁶ represents a hydrogen atom or an alkyl group having 1 to 6 carbon atoms; R³ is a group selected from any one of OH, OR⁴, NHR⁴, and NR⁴R⁵, and R⁴ and R⁵ are the same or different, and each is an unsubstituted alkyl group having 1 to 4 carbon atoms.]

## Description

### Technical Field

The present invention relates to, for example, an agent for preventing or ameliorating metabolic dysfunction-associated steatotic liver diseases (also referred to as "MASLD" in the present specification), such as metabolic dysfunction-associated steatohepatitis (also referred to as "MASH" in the present specification), the agent comprising a specific indole compound as an active ingredient.

### Background Art

The liver is the largest organ with 500 or more diverse functions. In addition to the function of reprocessing various nutrients absorbed in the intestines and supplying them to other organs while storing surplus, the liver also performs many functions necessary to maintain life, such as bile production and secretion, detoxification, and excretion. However, this organ is susceptible to damage from various factors, such as irregular lifestyle, stress, viruses, drugs, alcohol, malnutrition, and hepatic circulatory disorders, which can lead to acute hepatitis, chronic hepatitis, fatty liver, cirrhosis, and other diseases. In Japan, in particular, it is estimated that 15% or more of adults (an estimated 1.5 to 2 million people) have fatty liver, and its spread among younger generations is becoming a major social problem.

Fatty liver has been considered primarily a pathological condition associated with alcoholic liver disease caused by excessive alcohol consumption. Alcoholic fatty liver refers to a pathological condition of alcohol drinkers in which fat accumulation in the liver leads to the formation of fat droplets within liver cells, and further the deposition of fat (particularly triglycerides) in liver cells causes liver damage. However, in recent years, it has become clear that findings similar to alcoholic liver disease can also appear in individuals who do not consume alcohol.

Metabolic dysfunction-associated steatotic liver diseases (MASLD) are known as one of the pathological conditions of fatty liver damage in individuals who do not consume alcohol. MASLD is a disease which was previously called as a nonalcoholic fatty liver disease (NAFLD), and is also called a metabolic dysfunction-associated fatty liver disease (MAFLD). MASLD is found as fatty liver with deposition of triglycerides observed in liver cells; however, in approximately 90% of those patients, it is simple fatty liver having symptoms that can be improved with appropriate dietary therapy and exercise therapy.

However, it is known that approximately 10% of the MASLD cases are metabolic dysfunction-associated steatohepatitis (MASH), which presents a progressive pathological condition. MASH is a disease which was previously called nonalcoholic steatohepatitis (NASH). While there is no unified view on the histopathological progression of MASH, there is a certain consensus that steatohepatitis with inflammatory findings and fibrosis in liver tissue is observed when liver cells with accumulated fat undergo degeneration and necrosis. In addition, in subjects with multiple risk factors, such as metabolic syndrome, obesity, diabetes, hyperlipidemia, hypertension, and hyperuricemia, the probability of progression from fatty liver to MASH, further to more severe liver fibrosis, cirrhosis, and liver cancer, is considered to be higher.

On the other hand, the present inventors have reported that 4-(2,4-difluorophenyl)-2-(1H-indol-3-yl)-4-oxo-butanoic acid (i.e., MA5) has an effect of enhancing erythropoietin expression and a therapeutic effect on mitochondrial disease (Patent Document 1), an inhibitory effect on organ fibrosis (Patent Document 2), and an effect of preventing or improving hearing loss (Patent Document 3). However, it has not been known so far that the present compound has a therapeutic effect on MASLD.

### Prior Art Documents

### Patent Documents

Patent Document 1: International Publication No. WO 2014/080640 Pamphlet
Patent Document 2: Japanese unexamined Patent Application Publication No. 2015-189670
Patent Document 3: Japanese unexamined Patent Application Publication No. 2019-116453

### Summary of the Invention

### Object to be Solved by the Invention

An object of the present invention is to provide an agent for preventing or ameliorating MASLD, such as MASH, comprising, as an active ingredient, a low-molecular-weight compound that can be produced relatively easily and inexpensively.

### Means to Solve the Object

The present inventors are continuing extensive research to solve the above object. In the process, the present inventors have found that the present compound, described later, has the following effects: 1) an effect of improving weight gain and increased activity of blood aspartate aminotransferase (also referred to as "AST" in the present specification) and increased activity of blood alanine aminotransferase (also referred to as "ALT" in the present specification) caused by MASH, 2) an effect of improving inflammation and fibrosis in liver tissue caused by MASH, 3) an effect of improving increased blood AST activity and increased blood ALT activity caused by MASLD, 4) an effect of improving steatosis and fibrosis in liver tissue caused by MASLD, and 5) an effect of improving weight loss and shortened lifespan (due to fatty liver induced by decreased nutritional intake) caused by MASLD. Thus, the present invention has been completed.

Specifically, the present invention is as described below.
[1] An agent for preventing or ameliorating a metabolic dysfunction-associated steatotic liver disease, comprising one or more compounds selected from the group consisting of compounds of the following formula (Io) and physiologically acceptable salts thereof: 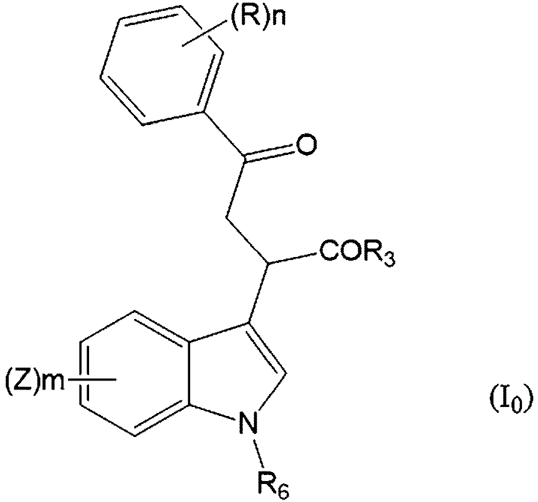 [wherein R is a halogen atom, an alkyl group having 1 to 7 carbon atoms, or an alkoxyl group having 1 to 7 carbon atoms, n is an integer of any one of 0 to 3; Z is a halogen atom, an alkyl group having 1 to 6 carbon atoms, an alkenyl group having 2 to 6 carbon atoms, an alkynyl group having 2 to 6 carbon atoms, or an organic oxy group of OR⁸, m is an integer of any one of 0 to 4, R⁸ represents an alkyl group having 1 to 7 carbon atoms, an alkenyl group having 2 to 6 carbon atoms, or an alkynyl group having 2 to 6 carbon atoms; when n and m are plural, each R and each Z are the same or different; R⁶ represents a hydrogen atom or an alkyl group having 1 to 6 carbon atoms; R³ is a group selected from any one of OH, OR⁴, NHR⁴, and NR⁴R⁵, and R⁴ and R⁵ are the same or different, and each is an unsubstituted alkyl group having 1 to 4 carbon atoms.]
[2] The agent according to [1] above, wherein the compound of formula (Io) is a compound of the following formula (I-1): 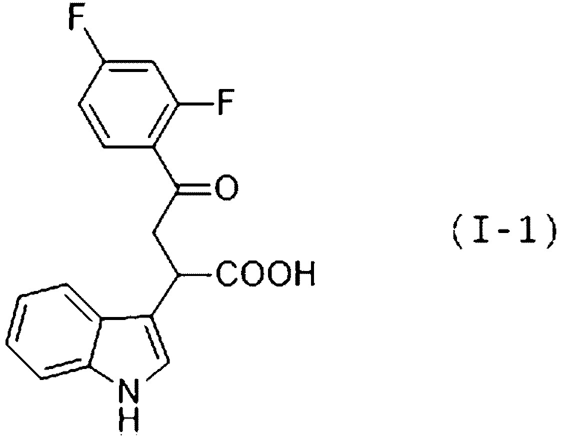
[3] The agent according to [1] or [2] above, wherein the metabolic dysfunction-associated steatotic liver disease is metabolic dysfunction-associated steatohepatitis.
[4] The agent according to any one of [1] to [3] above, wherein the agent is for oral ingestion.

Other embodiments of the present invention include:
a method for preventing or ameliorating (treating) a metabolic dysfunction-associated steatotic liver disease, comprising causing a mammal (e.g., a human, particularly a patient) in need of prevention or amelioration (treatment) of a metabolic dysfunction-associated steatotic liver disease to ingest (e.g., by administration) one or more compounds selected from the group consisting of compounds of the following formula (Io) and physiologically acceptable salts thereof (also referred to as "the present compound" in the present specification);
the present compound for use in the prevention or amelioration (treatment) of a metabolic dysfunction-associated steatotic liver disease; and
use of the present compound for producing an agent for preventing or ameliorating (treating) a metabolic dysfunction-associated steatotic liver disease.

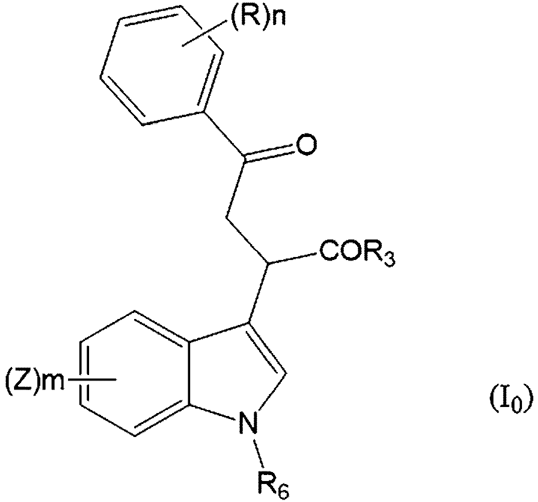

In the above formula (Io), R is a halogen atom, an alkyl group having 1 to 7 carbon atoms, or an alkoxyl group having 1 to 7 carbon atoms, n is an integer of any one of 0 to 3; Z is a halogen atom, an alkyl group having 1 to 6 carbon atoms, an alkenyl group having 2 to 6 carbon atoms, an alkynyl group having 2 to 6 carbon atoms, or an organic oxy group of OR⁸, m is an integer of any one of 0 to 4, R⁸ represents an alkyl group having 1 to 7 carbon atoms, an alkenyl group having 2 to 6 carbon atoms, or an alkynyl group having 2 to 6 carbon atoms; when n and m are plural, each R and each Z are the same or different; R⁶ represents a hydrogen atom or an alkyl group having 1 to 6 carbon atoms; R³ is a group selected from any one of OH, OR⁴, NHR⁴, and NR⁴R⁵, and R⁴ and R⁵ are the same or different, and each is an unsubstituted alkyl group having 1 to 4 carbon atoms.

### Effect of the Invention

According to the present invention, an agent for preventing or ameliorating metabolic dysfunction-associated steatotic liver diseases can be provided. Furthermore, the present invention allows for relatively easy and inexpensive production because a low-molecular-weight compound that can be produced relatively easily and in high yield is used as an active ingredient for the prevention or amelioration of metabolic dysfunction-associated steatotic liver diseases.

### Brief Description of Drawings

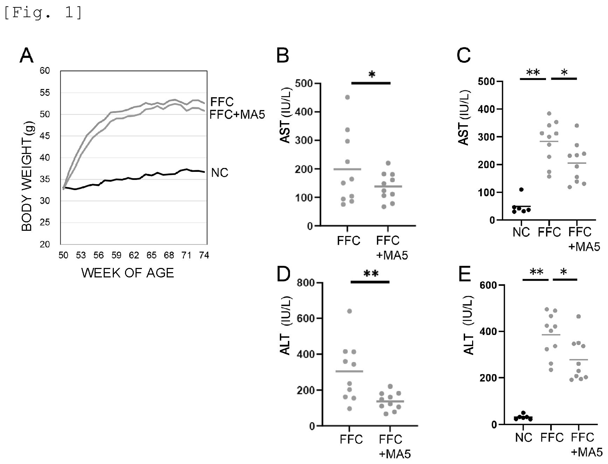

[Figure 1] Figure 1 shows the results of measuring mouse body weight (Figure 1A), blood AST activity after 15 weeks of intervention (Figure 1B), blood AST activity after 25 weeks of intervention (Figure 1C), blood ALT activity after 15 weeks of intervention (Figure 1D), and blood ALT activity after 25 weeks of intervention (Figure 1E) for three intervention groups (control group ["NC" in the figure], FFC group ["FFC" in the figure], and FFC+MA5 group ["FFC+MA5" in the figure]). In Figures 1B to 1E, **•** indicates individual mice, the bars indicate means, and "*" and "**" indicate statistically significant differences (p<0.05 and p<0.01, respectively).

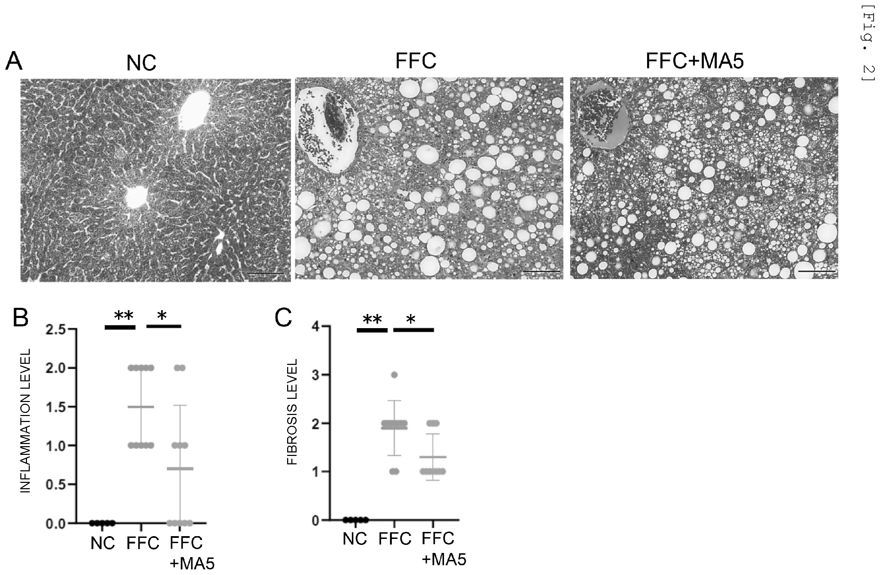

[Figure 2] Figure 2A shows microscopic images of mouse liver tissue after Masson's trichrome staining for three intervention groups (control group ["NC" in the figure], FFC group ["FFC" in the figure], and FFC+MA5 group ["FFC+MA5" in the figure]). Figure 2B shows the results of measuring inflammation levels in mouse liver tissue after Hematoxylin-Eosin (HE) staining on the mouse liver tissue in the three intervention groups. Figure 2C shows the results of measuring fibrosis levels in mouse liver tissue based on the microscopic images in Figure 2A. In Figures 2B and 2C, **•** indicates individual mice, the central bars indicate means, the bars at both ends indicate standard deviation (SD), and "*" and "**" indicate statistically significant differences (p<0.05 and p<0.01, respectively).

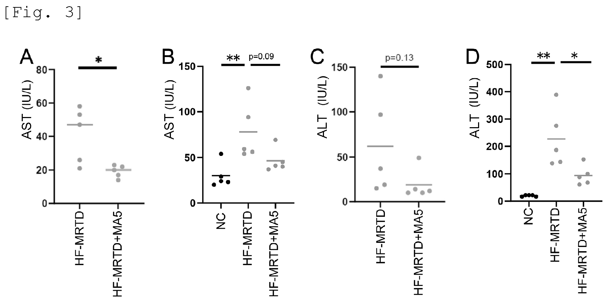

[Figure 3] Figure 3 shows the results of measuring blood AST activity after 13 weeks of intervention (Figure 3A), blood AST activity after 22 weeks of intervention (Figure 3B), blood ALT activity after 13 weeks of intervention (Figure 3C), and blood ALT activity after 22 weeks of intervention (Figure 3D) for three intervention groups (control group ["NC" in the figure], HF-MRTD group ["HF-MRTD" in the figure], and HF-MRTD+MA5 group ["HF-MRTD+MA5" in the figure]). In Figures 3A to D, **•** indicates individual mice, the bars indicate means, and "*" and "**" indicate statistically significant differences (p<0.05 and p<0.01, respectively).

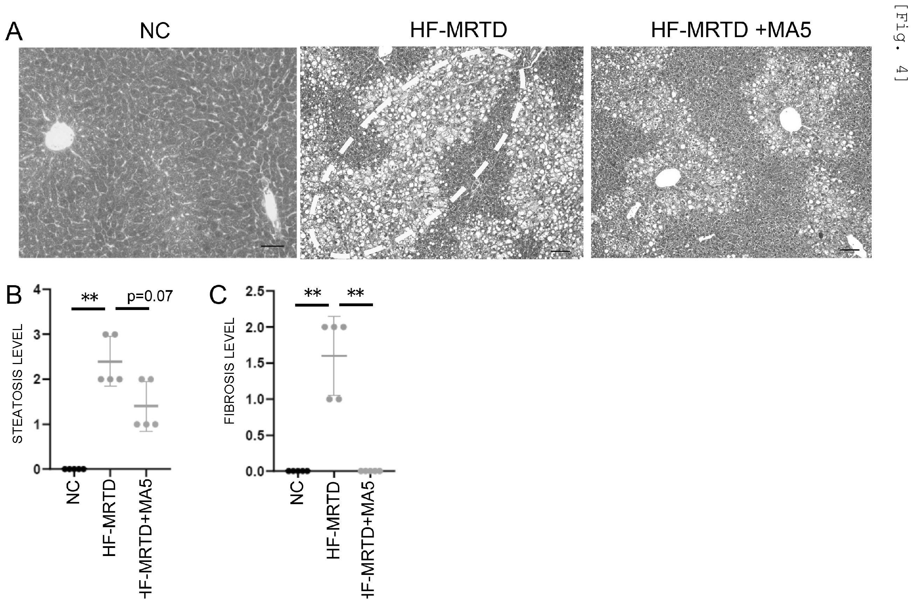

[Figure 4] Figure 4A shows microscopic images of mouse liver tissue after Masson's trichrome staining for three intervention groups (control group ["NC" in the figure], HF-MRTD group ["HF-MRTD" in the figure], and HF-MRTD+MA5 group ["HF-MRTD+MA5" in the figure]). The area enclosed by the dotted line indicates the region in which fibrosis was observed in the fat area. Figure 4B shows the results of measuring steatosis levels in mouse liver tissue after HE staining on the mouse liver tissue in the three intervention groups. Figure 4C shows the results of measuring fibrosis levels in mouse liver tissue based on the microscopic images in Figure 4A. In Figures 4B and 4C, **•** indicates individual mice, the central bars indicate means, the bars at both ends indicate standard deviation (SD), and "**" indicates a statistically significant difference (p<0.01).

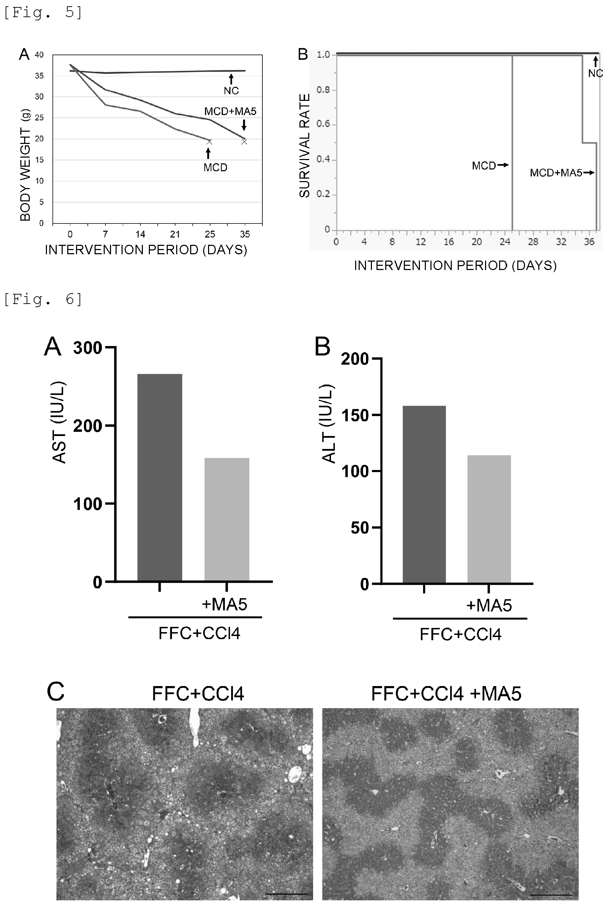

[Figure 5] Figure 5 shows the results of measuring the body weight (Figure 5A) and survival rate (Figure 5B) of mice for three intervention groups (control group ["NC" in the figure], MCD group ["MCD" in the figure], and MCD+MA5 group ["MCD+MA5" in the figure]).
[Figure 6] Figures 6A and 6B show the results of measuring blood AST activity (Figure 6A) and blood ALT activity (Figure 6B) for two intervention groups (advanced fibrosis group ["FFC+CCl4" in the figure] and advanced fibrosis+MA5 group ["FFC+CCl4+MA5" in the figure]). Figure 6C shows microscopic images of mouse liver tissue after Masson's trichrome staining for the two intervention groups.

### Modes of Carrying Out the Invention

The agent for preventing or ameliorating of the present invention is an agent comprising the present compound that is specifically designated for use in "preventing or ameliorating metabolic dysfunction-associated steatotic liver diseases" (hereinafter also referred to as "the present preventive/ameliorative agent").

The present compound, which is the active ingredient of the present agent, may be used alone as livestock feed, a food and beverage, a cosmetic, a quasi-drug, or a pharmaceutical (formulation), or may be further mixed with additives and used in the form of a composition (a livestock feed composition, a food and beverage composition, a cosmetic composition, a quasi-drug composition, or a pharmaceutical composition). Examples of the above food and beverage include a health food (e.g., a functional food, a nutritional supplement, a health supplement, a fortified food, a nutritional adjustment food, and a supplement) and a food with health claims (e.g., a food for specified health uses, a food with nutritional function claims, and a food with functional claims).

In the present specification, "metabolic dysfunction-associated steatotic liver disease" refers to fatty liver that is not caused by alcohol, and refers to, for example, a condition (pathological condition) in which triglyceride deposition in liver cells (generally triglyceride deposition in at least 5% or more [e.g., 5% or more, 10% or more, 15% or more, 20% or more, 25% or more, or 30% or more] of liver cells) is observed in liver tissue of a mammal with no history of alcohol consumption or a mammal with almost no history of alcohol consumption (e.g., when the mammal is a human, at least less than 20 g per day [e.g., less than 20 g per day, less than 25 g per day, or less than 30 g per day] in terms of ethanol).

In the present specification, "metabolic dysfunction-associated steatohepatitis" refers to a metabolic dysfunction-associated steatotic liver disease that exhibits a progressive pathological condition, and refers to a metabolic dysfunction-associated steatotic liver disease in which inflammation occurs in liver tissue. Metabolic dysfunction-associated steatohepatitis is a condition with one or more findings of steatohepatitis in liver tissue selected from, for example, deposition of large fat droplets, inflammatory cell infiltration, and balloon-like enlargement of liver cells; in some cases, it is accompanied by liver fibrosis or its progression. "Metabolic dysfunction-associated steatohepatitis" does not include hepatitis caused by factors other than nonalcoholic fatty liver (e.g., viral hepatitis and autoimmune hepatitis).

The metabolic dysfunction-associated steatotic liver diseases include not only metabolic dysfunction-associated steatohepatitis, but also pathological conditions that do not meet the definition of metabolic dysfunction-associated steatohepatitis but are considered to be in the process of transferring to MASH in which localized fatty degeneration and necrosis of the liver parenchyma or interstitial expansion or the like in the centrilobular and portal areas is observed, as well as conditions in which liver transaminases (e.g., AST and ALT) in the blood show abnormal activity (e.g., an increase of about twice or higher than that of healthy individuals), weight gain or loss, and shortened lifespan.

In the present specification, examples of the "mammal" include a human and a non-human mammal (e.g., a cow, a pig, sheep, a goat, a mouse, a rat, a hamster, and a guinea pig). Other examples of the non-human mammal include livestock (e.g., a cow, a pig, sheep, and a goat).

In the present specification, "preventing or ameliorating metabolic dysfunction-associated steatotic liver diseases" means one or more selected from the following: prevention of the onset of metabolic dysfunction-associated steatotic liver diseases; delayed onset of metabolic dysfunction-associated steatotic liver diseases; prevention or delay of the progression of severe conditions of metabolic dysfunction-associated steatotic liver diseases; improved level of severity of metabolic dysfunction-associated steatotic liver diseases; and remission of metabolic dysfunction-associated steatotic liver diseases.

The compound included in the present compound is not particularly limited as long as it is one or more compounds selected from the group consisting of compounds of the following formula (Io) and physiologically acceptable salts thereof.

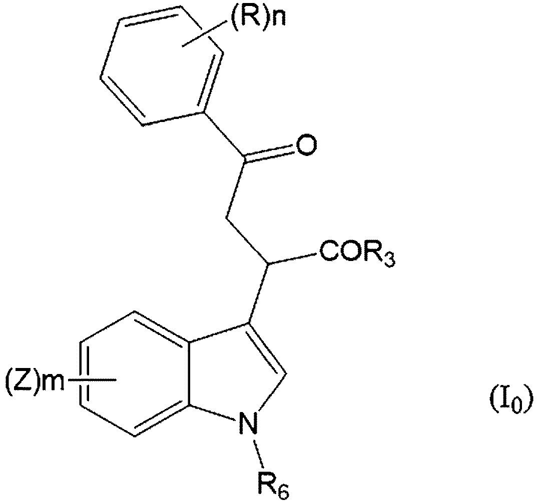

In the above formula (Io),
R is a halogen atom, an alkyl group having 1 to 7 carbon atoms, or an alkoxyl group having 1 to 7 carbon atoms.

n is an integer of any one of 0 to 3.

Z is a halogen atom, an alkyl group having 1 to 6 carbon atoms, an alkenyl group having 2 to 6 carbon atoms, an alkynyl group having 2 to 6 carbon atoms, or an organic oxy group of OR⁸.

m is an integer of any one of 0 to 4.

R⁸ represents an alkyl group having 1 to 7 carbon atoms, an alkenyl group having 2 to 6 carbon atoms, or an alkynyl group having 2 to 6 carbon atoms.

When n and m are plural, each R and each Z are the same or different.

R⁶ represents a hydrogen atom or an alkyl group having 1 to 6 carbon atoms.

R³ is a group selected from any one of OH, OR⁴, NHR⁴, and NR⁴R⁵.

R⁴ and R⁵ are the same or different, and each is an unsubstituted alkyl group having 1 to 4 carbon atoms.

(R)ₙ in the compound of the above formula (Io) means unsubstituted when n is 0, and when n is an integer of any one of 1 to 3, it means that 1 to 3 of the hydrogen atoms bonded to the carbon atoms constituting the benzene ring are each replaced by a halogen atom, an alkyl group having 1 to 7 carbon atoms (C), or an alkoxyl group having 1 to 7 carbon atoms. When n is 2 or 3 (i.e., when the substituted benzene ring has 2 or 3 substituents), the substituents are the same or different. Examples of (R)ₙ in the compound of the above formula (Io) include 1 to 3 halogen atoms, 1 to 3 alkyl groups having 1 to 7 carbon atoms, and 1 to 3 alkoxyl groups having 1 to 7 carbon atoms.

(Z)ₘ in the compound of the above formula (Io) means unsubstituted when m is 0, and when m is an integer of any one of 1 to 4, it means that 1 to 4 of the hydrogen atoms bonded to the carbon atoms constituting the benzene ring are each replaced by a halogen atom, an alkyl group having 1 to 6 carbon atoms, an alkenyl group having 2 to 6 carbon atoms, an alkynyl group having 2 to 6 carbon atoms, or an organic oxy group of OR⁸ (wherein R⁸ represents an alkyl group having 1 to 7 carbon atoms, an alkenyl group having 2 to 6 carbon atoms, or an alkynyl group having 2 to 6 carbon atoms). When m is an integer of any one of 2 to 4 (i.e., when the substituted benzene ring has 2 to 4 substituents), the substituents are the same or different. Examples of (Z)ₘ in the compound of the above formula (Io) include 1 to 3 halogen atoms, 1 to 3 alkyl groups having 1 to 6 carbon atoms, 1 to 3 alkenyl groups having 2 to 6 carbon atoms, 1 to 3 alkynyl groups having 2 to 6 carbon atoms, and 1 to 3 organic oxy groups of OR⁸.

The "alkyl group having 1 to 7 carbon atoms" in the above formula (Io) may be a linear or branched alkyl group, and examples thereof include a methyl group, an ethyl group, a propyl group, a 2-ethylbutyl group, a 2-methylpentyl group, and a 3-methylpentyl group, with a methyl group, a 2-ethylbutyl group, a 2-methylpentyl group, or a 3-methylpentyl group being preferred.

The "alkoxyl group having 1 to 7 carbon atoms" in the above formula (Io) may be a linear or branched alkoxyl group, and examples thereof include a methoxy group, an ethoxy group, and a propoxy group.

The "alkyl group having 1 to 6 carbon atoms" in the above formula (Io) refers to a linear or branched alkyl group having 1 to 6 carbon atoms optionally having a substituent, and the alkyl group having 1 to 6 carbon atoms is preferably an alkyl group having 1 to 3 carbon atoms, such as a methyl group, an ethyl group, an n-propyl group, or an isopropyl group, or a benzyl group.

The "alkenyl group having 2 to 6 carbon atoms" in the above formula (Io) refers to a linear or branched alkenyl group having 2 to 6 carbon atoms optionally having a substituent, and examples thereof include an ethenyl group (vinyl group), a 1-propenyl group, and a 2-propenyl group (allyl group).

The "alkynyl group having 2 to 6 carbon atoms" in the above formula (Io) refers to a linear or branched alkynyl group having 2 to 6 carbon atoms optionally having a substituent, and examples thereof include an ethynyl group, a 1-propynyl group, a 1-butynyl group, a 1-pentynyl group, and a 1-hexynyl group.

Examples of the "halogen atom" in the above formula (Io) include a fluorine atom, a chlorine atom, a bromine atom, and an iodine atom.

Examples of the "unsubstituted alkyl group having 1 to 4 carbon atoms" in the above formula (Io) include a methyl group, a monochloromethyl group, an ethyl group, a 1-phenylethyl group, a 2-phenylethyl group, a 2-methoxyethyl group, a 2,2,2-trichloroethyl group, a propyl group, an isopropyl group, a hexafluoroisopropyl group, an n-butyl group, an isobutyl group, a sec-butyl group, a tert-butyl group, and pyrrolidine in which R⁴ and R⁵ are taken together with nitrogen.

When the present compound has a chiral carbon atom and a stereogenic center associated with axial chirality, the compound includes all possible optical isomers, and those optical isomers can be used in any ratio. For example, a certain optically active compound can be used as an enantiomer, a racemate, or a mixture of enantiomers in any ratio. When there are multiple stereogenic centers, it may also be used as a mixture of diastereomers in any ratio.

The physiologically acceptable salts of the compound of the above formula (Io) include metal salts formed from aluminum, calcium, lithium, magnesium, potassium, sodium, and zinc; and organic salts formed from N,N'-dibenzylethylenediamine, chloroprocaine, choline, diethanolamine, ethylenediamine, N-methylglucamine, lysine, procaine, and the like.

The method for synthesizing the present compound can be any of the methods disclosed in Patent Document 1, International Publication No. WO 2019/235455 Pamphlet, and the like, but is not limited to these methods, and generally known synthesis methods can be used.

The compound of the above formula (Io) is preferably a compound of the following formula (I-1) (i.e., a compound of the above formula (Io) wherein R is a fluorine atom, n is 2, m is 0, R⁶ is a hydrogen atom, and R³ is OH) (also referred to as "MA5" in the present specification) because its effect has been demonstrated in the Examples of the present specification.

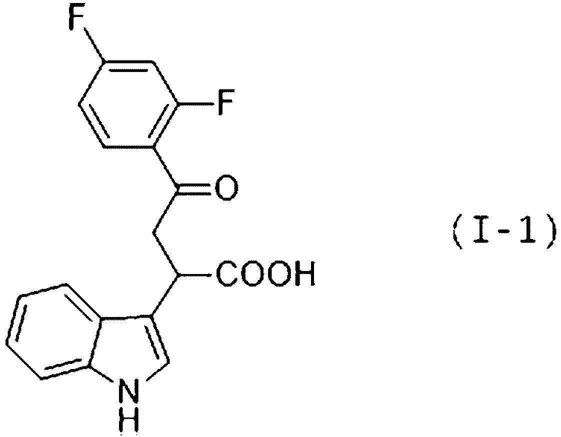

Examples of additives for the present preventive/ameliorative agent include general physiologically acceptable carriers, binders, stabilizers, excipients, diluents, pH buffers, disintegrants, isotonic agents, coatings, solubilizers, lubricants, gliding agents, dissolution aids, glidants, flavoring agents, sweeteners, solvents, gelling agents, nutrients, various oiling agents, surfactants, preservatives, antioxidants, dispersants, chelating agents, thickeners, UV absorbers, emulsifying stabilizers, pH adjusters, colorants, fragrances, and other ingredients. Specific examples of such ingredients include water, physiological saline, animal fats and oils, vegetable oils, lactose, starch, gelatin, crystalline cellulose, gum, talc, magnesium stearate, hydroxypropylcellulose, polyalkylene glycol, polyvinyl alcohol, and glycerol.

The subject to whom the present preventive/ameliorative agent should be ingested (e.g., administered) may be any subject (mammal) in need of prevention or amelioration of metabolic dysfunction-associated steatotic liver diseases. Preferred subjects include those at risk of suffering metabolic dysfunction-associated steatotic liver diseases, or those who have suffered metabolic dysfunction-associated steatotic liver diseases (MASLD patients), and more preferred subjects include those at risk of suffering metabolic dysfunction-associated steatohepatitis, or those who have suffered metabolic dysfunction-associated steatohepatitis (MASH patients). These individuals may be elderly people (e.g., 60 years or older, 65 years or older, 70 years or older, 75 years or older, or 80 years or older), and further may be individuals (cancer patients) suffering from cancer (e.g., liver cancer).

The method of causing ingestion (e.g., by administration) of the present preventive/ameliorative agent may be any method by which the present compound contained in the present preventive/ameliorative agent is incorporated into the body of a mammal. The method may be an oral ingestion method (e.g., oral administration in a dosage form, such as a powder, a granule, a tablet, a capsule, or a syrup) or a parenteral administration method (e.g., parenteral administration in a dosage form, such as a topical skin preparation, a transdermal preparation, a transmucosal preparation, a nasal preparation, an enteral preparation, an injection, a suppository, an inhalant, or a patch); however, an oral ingestion method can be suitably exemplified because its effect has been demonstrated in the Examples described later.

The amount of the present preventive/ameliorative agent or the present compound contained in the present preventive/ameliorative agent to be ingested is appropriately determined depending on the type, age, body weight, sex, symptoms, drug sensitivity, and the like of the mammal for ingestion (e.g., for administration). For example, the amount to be ingested is within the range of 1 µg to 200 mg/kg (body weight)/day (e.g., 500 µg/kg/day to 100 mg/kg/day, 1 mg/kg/day to 50 mg/kg/day, or 3 mg/kg/day to 25 mg/kg/day). Moreover, the present preventive/ameliorative agent is ingested once or several times (e.g., 2 to 4 times) a day; however, the amount to be ingested may be adjusted according to the degree of effect.

The present preventive/ameliorative agent may also contain, other than the present compound, substances that have a preventive or ameliorative effect on metabolic dysfunction-associated steatotic liver diseases (e.g., compounds, proteins [e.g., antibodies], DNA, and RNA); however, since the present compound alone exhibits an excellent preventive or ameliorative effect on metabolic dysfunction-associated steatotic liver diseases, it is preferable that the present preventive/ameliorative agent does not contain, other than the present compound, substances that have a preventive or ameliorative effect on metabolic dysfunction-associated steatotic liver diseases.

The present invention will be described in more detail below with reference to Examples; however, the technical scope of the present invention is not limited to these examples.

### Examples

### 1. Therapeutic effect on FFC diet model

In order to confirm that the present compound is effective for the treatment of MASLD, analyses were performed using a high-fructose, high-saturated fatty acid, high-cholesterol diet (FFC diet) model, that is, diet-induced MASH model mise.

### 1-1. Method

After 49-week-old C57BL/6J mice (obtained from Japan SLC, Inc.) (male, n=16) and 50-week-old C57BL/6J mice (obtained from CLEA Japan, Inc.) (male, n=10) were acclimatized for one week, they were randomly divided into three intervention groups (control group [n=6], FFC group [n=10], and FFC+MA5 group [n=10]) and subjected to intervention for 25 weeks. The control group was fed a standard diet (CE-2, manufactured by CLEA Japan, Inc.) and water, the FFC group was fed a high-fat, high-cholesterol diet (Western Diet powder [manufactured by Research Diets, Inc., D12079BM]) and high-glucose, high-fructose water (18.9 g D-glucose/L, 23.1 g D-fructose/L), and the FFC+MA5 group was fed a diet obtained by mixing the above high-fat, high-cholesterol diet with MA5 in a mass ratio of 0.012% (equivalent to approximately 5 mg/kg/day of MA5 when converted to a human dosage based on the conversion factor per body surface area) and the above high-glucose, high-fructose water. The mice were weighed and the various diets and water were replenished once a week. After 15 weeks of intervention (i.e., at 64 to 65 weeks of age), the mice in each group were anesthetized, and approximately 200 µL of blood samples were collected from the orbital venous plexus. After 25 weeks of intervention (i.e., at 74 to 75 weeks of age), the mice were euthanized after 4 hours of fasting, and blood samples and liver tissue were rapidly collected. After plasma separation, the blood samples were measured for blood AST activity and blood ALT activity using Spotchem II Kenshin-2 (manufactured by ARKRAY, Inc.). Based on the collected liver tissue, formalin-fixed, paraffin-embedded specimens were prepared and subjected to HE staining, and inflammation levels in the liver tissue were measured using an optical microscope based on the NAFLD activity score (NAS). Further, Masson's trichrome staining was performed, and fibrosis levels in the liver tissue were measured using an optical microscope according to the method described in the document "Hepatology. 2005 Jun;41(6):1313-21."

### 1-2. Results

As a result of measuring mouse body weight, blood AST activity, and blood ALT activity for the three intervention groups (control group, FFC group, and FFC+MA5 group), the FFC group showed an increase in mouse body weight (Figure 1A) and significant increases in blood AST activity and blood ALT activity (Figures 1C and 1E) compared to the control group. In contrast, compared to the FFC group, the FFC+MA5 group showed a reduction in the increased body weight (Figure 1A) and a significant reduction in the increased blood AST activity and blood ALT activity (Figures 1C and 1E), and it was demonstrated that these effects were maintained over at least 15 to 25 weeks (Figures 1B to E).

These results indicate that MA5 has an effect of improving weight gain and increased blood AST activity and blood ALT activity caused by MASH.

In addition, as a result of analyzing inflammation and fibrosis levels in mouse liver tissue for the above three intervention groups, the FFC group showed inflammation and fibrosis in liver tissue, which were not observed in the control group, whereas the FFC+MA5 group showed significant decreases in the increased inflammation and fibrosis levels (Figure 2) compared to the FFC group.

These results indicate that MA5 has an effect of improving inflammation and fibrosis in liver tissue caused by MASH.

### 2. Therapeutic effect on HF-MRTD diet model

In order to confirm that the present compound is effective for the treatment of MASLD, analyses were performed using a high-fat, methionine-restricted, tyrosine-deficient diet (HF-MRTD diet) model, that is, diet-induced MASLD model mice.

### 2-1. Method

After 6-week-old C57BL/6J mice (obtained from Japan SLC, Inc.) (male, n=15) were acclimatized for one week, they were randomly divided into three intervention groups (control group [n=5], HF-MRTD group [n=5], and HF-MRTD+MA5 group [n=5]) and subjected to intervention for 22 weeks. The control group was fed a standard diet (CE-2, manufactured by CLEA Japan, Inc.) and water, the HF-MRTD group was fed a powdered MRTD diet (a diet containing 60% fat by calorie and 0.2% methionine by mass, prepared according to the method described in the document "Hepatology. 2021 Sep;74(3):1271-1286.") and high-glucose, high-fructose water (18.9 g D-glucose/L, 23.1 g D-fructose/L), and the HF-MRTD+MA5 group was fed a diet obtained by mixing the above powdered MRTD diet with MA5 in a mass ratio of 0.012%, and the above high-glucose, high-fructose water. The various diets and water were replenished once a week. After 13 weeks of intervention (i.e., at 19 weeks of age), the mice in each group were anesthetized, and approximately 200 µL of blood samples were collected from the orbital venous plexus. After 22 weeks of intervention (i.e., at 28 weeks of age), the mice were euthanized after 4 hours of fasting, and blood samples and liver tissue were rapidly collected. After plasma separation, the blood samples were measured for blood AST activity and blood ALT activity using Spotchem II Kenshin-2 (manufactured by ARKRAY, Inc.). Based on the collected liver tissue, formalin-fixed, paraffin-embedded specimens were prepared and subjected to HE staining, and steatosis levels in liver tissue were measured using an optical microscope based on NAS. Further, Masson's trichrome staining was performed, and fibrosis levels in the liver tissue were measured using an optical microscope according to the method described in the document "Hepatology. 2005 Jun;41(6):1313-21."

### 2-2. Results

As a result of measuring blood AST activity and blood ALT activity for the three intervention groups (control group, HF-MRTD group, and HF-MRTD+MA5 group), the FFC group showed significant increases in blood AST activity and blood ALT activity (Figures 3B and 3D) compared to the control group, whereas the FFC+MA5 group showed a reduction in the increased blood AST activity (Figure 3B) and a significant reduction in the increased blood ALT activity (Figure 3D) compared to the FFC group, and it was demonstrated that these effects were maintained over at least 13 to 22 weeks (Figures 3A to D).

These results indicate that MA5 has an effect of improving increased blood AST activity and blood ALT activity caused by MASLD.

In addition, as a result of analyzing steatosis and fibrosis levels in mouse liver tissue for the above three intervention groups, the HF-MRTD group showed steatosis and fibrosis in liver tissue, which were not observed in the control group, whereas the HF-MRTD+MA5 group showed a decrease in the increased steatosis level (Figure 4B) and a significant decrease in the increased fibrosis level (Figures 4A and 4C), compared to the HF-MRTD group.

These results indicate that MA5 has an effect of improving steatosis and fibrosis in liver tissue caused by MASLD.

### 3. Therapeutic effect on MCD diet model

In order to confirm that the present compound is effective for the treatment of MASLD, analyses were performed using a methionine- and choline-deficient diet (MCD diet) model, that is, diet-induced MASLD model mice.

### 3-1. Method

After 54-week-old C57BL/6J mice (obtained from Japan SLC, Inc.) (male, n=6) were acclimatized for one week, they were randomly divided into three intervention groups (control group [n=2], MCD group [n=2], and MCD+MA5 group [n=2]) and subjected to intervention until the individual mice died. The control group was fed a standard diet (CE-2, manufactured by CLEA Japan, Inc.) and water, the MCD group was fed a methionine- and choline-deficient diet (manufactured by Research Diets, Inc., A02082002BRM) and water, and the MCD+MA5 group was fed a diet obtained by mixing the above methionine- and choline-deficient diet with MA5 in a mass ratio of 0.012%, and water. During the period until the individual mice died, the mice were weighed and the various diets and water were replenished once a week.

### 3-2. Results

Compared to the control group, the MCD group showed a decrease in mouse body weight and a shortened mouse lifespan, whereas the MCD+MA5 group showed an increase in the decreased mouse body weight and an extension of the shortened mouse lifespan, compared to the MCD group (Figure 5).

These results indicate that MA5 has an effect of improving weight loss and shortened lifespan caused by MASLD (fatty liver induced by decreased nutritional intake).

### 4. Therapeutic effect on diet- and drug-induced MASH model mice with advanced fibrosis

In order to confirm that the present compound is effective for the treatment of MASLD, analyses were performed using diet- and drug-induced MASH model mice with advanced fibrosis.

### 4-1. Method

After 12-week-old C57BL/6J mice (obtained from Japan SLC, Inc.) (male, n=2) were acclimatized for one week, they were randomly divided into two intervention groups (advanced fibrosis group [n=1] and advanced fibrosis+MA5 group [n=1]) and subjected to intervention for 6 weeks. In order to create MASLD model mice with advanced fibrosis for these two intervention groups, the mice were fed a high-fat, high-cholesterol diet (Western Diet powder [manufactured by Research Diets, Inc., D12079BM]) and high-glucose, high-fructose water (18.9 g D-glucose/L, 23.1 g D-fructose/L), and carbon tetrachloride, which is a drug that induces liver damage and liver fibrosis, was continuously administered intraperitoneally. Further, the advanced fibrosis group was orally administered with DMSO three times a week using a feeding tube, and the advanced fibrosis+MA5 group was orally administered with MA5 (25 mg/kg) three times a week using a feeding tube. The diet and water were replenished once a week. After 6 weeks of intervention, the mice were euthanized after 4 hours of fasting, and blood samples and liver tissue were rapidly collected. After plasma separation, the blood samples were measured for blood AST activity and blood ALT activity using Spotchem II Kenshin-2 (manufactured by ARKRAY, Inc.). Based on the collected liver tissue, formalin-fixed, paraffin-embedded specimens were prepared and subjected to Masson's trichrome staining, and fibrosis levels in the liver tissue were observed using an optical microscope.

### 4-2. Results

As a result of measuring blood AST activity and blood ALT activity for the two intervention groups (advanced fibrosis group and advanced fibrosis+MA5 group), the advanced fibrosis group showed increased blood AST activity and blood ALT activity compared to the control group shown in Figures 1C and 1E, whereas the advanced fibrosis+MA5 group showed reductions in the increased blood AST activity and blood ALT activity (Figures 6A and 6B) compared to the advanced fibrosis group.

These results indicate that MA5 has an effect of improving increased blood AST activity and blood ALT activity caused by MASH with advanced fibrosis.

In addition, as a result of analyzing fibrosis levels in mouse liver tissue for the above two intervention groups, the advanced fibrosis group showed a level of fibrosis close to cirrhosis (corresponding to F3 to F4), which was not observed in the control group shown in Figure 4A. In contrast, in the advanced fibrosis+MA5 group, although fibrosis itself was observed, its progression was suppressed, corresponding to F1 (Figure 6C).

These results indicate that MA5 has an effect of improving fibrosis in liver tissue caused by MASH with advanced fibrosis.

### Industrial Applicability

The present invention contributes to the prevention or amelioration of MASLD.

## Claims

1. An agent for preventing or ameliorating a metabolic dysfunction-associated steatotic liver disease, comprising one or more compounds selected from the group consisting of compounds of the following formula (Io) and physiologically acceptable salts thereof: 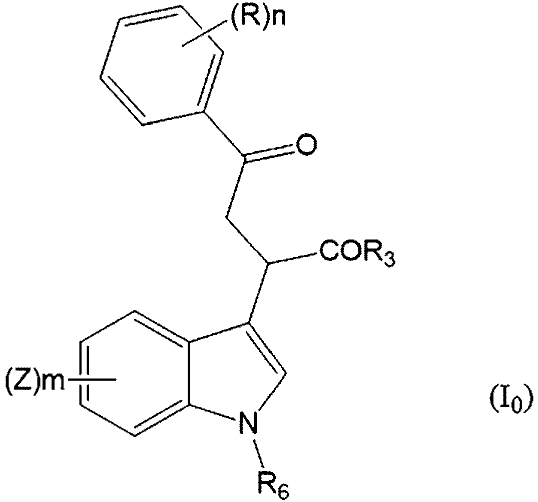 [wherein R is a halogen atom, an alkyl group having 1 to 7 carbon atoms, or an alkoxyl group having 1 to 7 carbon atoms, n is an integer of any one of 0 to 3; Z is a halogen atom, an alkyl group having 1 to 6 carbon atoms, an alkenyl group having 2 to 6 carbon atoms, an alkynyl group having 2 to 6 carbon atoms, or an organic oxy group of OR⁸, m is an integer of any one of 0 to 4, R⁸ represents an alkyl group having 1 to 7 carbon atoms, an alkenyl group having 2 to 6 carbon atoms, or an alkynyl group having 2 to 6 carbon atoms; when n and m are plural, each R and each Z are the same or different; R⁶ represents a hydrogen atom or an alkyl group having 1 to 6 carbon atoms; R³ is a group selected from any one of OH, OR⁴, NHR⁴, and NR⁴R⁵, and R⁴ and R⁵ are the same or different, and each is an unsubstituted alkyl group having 1 to 4 carbon atoms.]

2. The agent according to claim 1, wherein the compound of formula (Io) is a compound of the following formula (I-1):

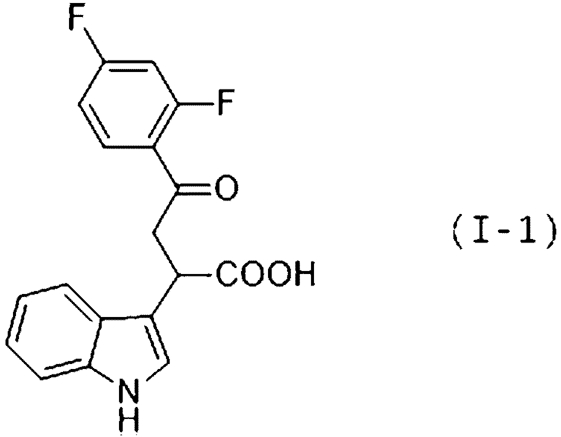

3. The agent according to claim 1 or 2, wherein the metabolic dysfunction-associated steatotic liver disease is metabolic dysfunction-associated steatohepatitis.

4. The agent according to claim 1 or 2, wherein the agent is for oral ingestion.
